Europäisches Patentamt

European Patent Office

Office européen des brevets

(11) Veröffentlichungsnummer: **0 129 014**
**A1**

(12) # EUROPÄISCHE PATENTANMELDUNG

(21) Anmeldenummer: **84104191.6**

(22) Anmeldetag: **13.04.84**

(51) Int. Cl.³: **A 61 K 7/32**

(30) Priorität: **04.06.83 DE 3320304**

(43) Veröffentlichungstag der Anmeldung:
**27.12.84 Patentblatt 84/52**

(84) Benannte Vertragsstaaten:
**AT BE CH DE FR GB IT LI NL SE**

(71) Anmelder: **Beiersdorf Aktiengesellschaft**
**Unnastrasse 48**
**D-2000 Hamburg 20(DE)**

(72) Erfinder: **Fleck, Wolfgang, Dipl.-Chem.Dr.**

**Deceased(DE)**

(72) Erfinder: **Hoppe, Udo, Dipl.-Chem. Dr.**
**Lottbeker Weg 7**
**D-2000 Hamburg 65(DE)**

(72) Erfinder: **Klier, Manfred, Dipl.-Chem. Dr.**
**Emil-Specht-Allee 11**
**D-2055 Aumühle(DE)**

(54) **Desodorierendes kosmetisches Mittel.**

(57) Die Erfindung betrifft ein desodorierendes kosmetisches Mittel auf Basis üblicher kosmetischer Trägerstoffe mit einem Gehalt an Wollwachssäuren oder an Alkali- oder Ammoniumsalzen von Wollwachssäuren. Dieses Mittel zeigt bei seiner Anwendung neben einer ausgezeichneten Haut- und Schleimhautverträglichkeit einen sehr guten Desodorierungseffekt.

EP 0 129 014 A1

BEIERSDORF AKTIENGESELLSCHAFT
HAMBURG

Desodorierendes kosmetisches Mittel

Die Erfindung betrifft ein desodorierendes kosmetisches Mittel auf Basis üblicher kosmetischer Trägerstoffe in den für derartige Präparate üblichen Mengenverhältnissen zur örtlichen Anwendung auf der Haut, das als Wirkstoff Wollwachssäuren oder deren Alkali- oder Ammoniumsalze enthält.

Desodorierende kosmetische Mittel, die insbesondere zum Unterdrücken von unangenehmem Körpergeruch eingesetzt werden, der durch Einwirkung von Bakterien auf den zunächst weitgehend geruchlosen apokrinen Schweiß unter dem Einfluß von Wärme und Feuchtigkeit infolge Bildung von stark riechenden Zersetzungsprodukten entsteht, sind seit Jahren in Form von Cremes, Stiften, Roll-on-Präparaten sowie als aus Aerosolbehältern versprühbare Sprays im Handel. Neben desodorierend wirkenden Stiften, die im Unterschied zu vielen anderen kosmetischen Anwendungsformen risikolos in Hand- und Badetaschen aufbewahrt und mitgeführt werden können, haben vor allem aus Aerosolbehältern versprühbare Deo-Produkte sowie Roll-on-Präparate wegen ihrer bequemen Handhabungsmöglichkeit bei den Verbrauchern eine zunehmende Bedeutung erlangt.

Um die unerwünschte bakterielle Zersetzung des Schweißes zu verhindern, enthalten desodorierende kosmetische Präparate als Wirkstoff geringe Mengen von antimikrobiell wirkenden Verbindungen, die eine Abtötung der für die Zersetzung des Schweißes verantwortlichen Hautbakterien bzw. eine Wachstumshemmung der bakteriellen Hautflora bewirken sollen. Für diesen Zweck wurde bereits eine Vielzahl unterschiedlicher Substanzen vorgeschlagen und verwendet, z.B. halogenierte Phenole, wie Hexachlorphenol, quaternäre Ammoniumverbindungen, wie Alkyldimethylbenzylammoniumchlorid, Cetyltrimethylammoniumbromid, p-Hydroxybenzoesäureester, 2,4,4'-Trichlor-2'-hydroxydiphenyläther und Undecylensäure-polydiäthanolamid.

Eine weitere Möglichkeit zur Schweißbekämpfung und damit zur Unterdrückung von unangenehmem Körpergeruch besteht indirekt durch Verminderung der Abgabe von Körperschweiß an die Hautoberfläche infolge Kontraktion der örtlichen Blutgefäße durch Anwendung von Antitranspirantien, d.h. von kosmetischen Mitteln, die bestimmte adstringierend wirkende Aluminium-, Zirkonium- oder Zinksalze enthalten.

Beide Arten von kosmetischen Präparaten haben jedoch bei längerdauernder örtlicher Anwendung auf bestimmten Hautbereichen erhebliche Nachteile.

So kann der längere Gebrauch von Antitranspirantien zu Hautreizungen und Hautveränderungen führen. Bei den kosmetischen Mitteln mit desodorierender Wirkung, die einen Gehalt an antimikrobiellen Substanzen aufweisen, besteht andererseits bei langdauernder Verwendung die Tendenz zu einer unerwünschten Verschiebung der Bakterienflora der Hautoberfläche und somit zur Störung des biologischen Gleichgewichts auf der Haut sowie zur Bildung resistenter Bakterien-Stämme. Außerdem kann es zu Lichtsensibilisierungen und toxischen Nebenwirkungen von unterschiedlicher Stärke kommen.

Aufgabe der Erfindung war daher die Schaffung eines desodorierenden kosmetischen Mittels, das die zuvor aufgezeigten Nachteile nicht aufweist, gut haut- und schleimhautverträglich und praktisch nicht toxisch ist und das schon bei niedrigen Konzentrationen an antimikrobiellem Wirkstoff sehr gut desodorierend wirkt.

Es wurde gefunden und darin liegt die Lösung der Aufgabe gemäß der Erfindung, daß man unter Verwendung der üblichen kosmetischen Trägerstoffe zu desodorierenden kosmetischen Mitteln mit sehr guten desodorierenden Eigenschaften, die sämtliche der oben angeführten Bedingungen erfüllen, dann gelangt, wenn man derartigen Mitteln geringe Mengen an Wollwachssäuren oder Alkali- oder Ammoniumsalzen von Wollwachssäuren zusetzt.

Gegenstand der Erfindung ist somit ein desodorierendes kosmetisches Mittel auf Basis üblicher kosmetischer Trägerstoffe sowie ein oder mehrerer Wirkstoffe, das dadurch gekennzeichnet ist, daß es als Wirkstoff Wollwachssäuren oder Alkali- oder Ammoniumsalze von Wollwachssäuren enthält.

Unter den Begriff "Wollwachssäuren" wird das bei der Verseifung von Wollwachs (Wollfett) mit äthanolischem Alkali nach Abtrennung der Wollwachsalkohole mit einem organischen Lösungsmittel und Ansäuern der Seifen in Form einer gelbbräunlichen klebrigen Masse von wachsartiger Konsistenz anfallende Gemisch von freien Säuren unterschiedlicher Art verstanden, die obwohl Glieder von vier verschiedenen homologen Reihen sämtlich der aliphatischen Gruppe angehören. Diese Rohsäuren werden - um für die erfindungsgemäße Verwendung geeignet zu sein - zur Reinigung und Abtrennung unerwünschter Substanzen und Nebenprodukte einer Destillation im Hochvakuum oder einer Molekulardestillation und daran anschließend einem Desodorierungsprozeß unterworfen. Dabei werden reine Wollwachssäuren als weißlich-gelbes Produkt von angenehmem Geruch erhalten, die als solche oder in Form ihrer in bekannter Weise daraus hergestellten Alkali- oder Ammoniumsalze für den erfindungsgemäßen Zweck verwendet werden können. Von den Alkalisalzen wird das Natriumsalz der Wollwachssäuren bevorzugt. Dieses kann entweder als solches den kosmetischen Zubereitungen zugesetzt oder aber dadurch erzeugt werden, das man in einem getrennten Arbeitsgang die reinen Wollwachssäuren vorlegt und diese mit einem aliquoten Teil einer Lösung aus einem Teil Ätznatron (Plätzchenform) und zwei Teilen Wasser bei 40°C etwa 20 Minuten lang verseift. Diese verseifte Mischung kann dann - unter Berücksichtigung der Gesamtwassermenge der Rezeptur - unmittelbar der Zubereitung zugefügt werden.

Da die Wollwachssäuren bei der Verseifung des Wollwachses zur Gewinnung der als Emulgator vom Typ W/O sehr begehrten Wollwachsalkohole in erheblicher Menge als Nebenprodukt anfallen, für das man bisher trotz großer Bemühungen keine rechte Verwendung gefunden hat, stellt die Verwendung gemäß der Erfindung einen preisgünstigen Weg für eine nutzbringende Verwendung der Wollwachssäuren dar.

Vorzugsweise soll der Gehalt des erfindungsgemäßen desodorierenden kosmetischen Mittels an Wollwachssäuren oder deren Alkali- oder Ammoniumsalzen 0,3 bis 6,0 Gewichts-%, bezogen auf die Gesamt-Zusammensetzung des Mittels, betragen.

Das desodorierende kosmetische Mittel gemäß der Erfindung kann in Form eines aus Aerosolbehältern, Quetschflaschen oder durch eine Pumpvorrichtung versprühbaren Präparates vorliegen oder in Form einer mittels Roll-on-Vorrichtungen auftragbaren flüssigen Zusammensetzung. Daneben jedoch aus einer aus normalen Flaschen und Behältern auftragbaren Lotion.

Als besonders zweckmäßig hat es sich erwiesen, bei den aus Aerosolbehältern versprühbaren Präparaten (Sprays) einen Gehalt an Wollwachssäuren bzw. Alkali- oder Ammoniumsalzen von Wollwachssäuren von 0,5 bis 1,5 Gewichts-% zu wählen; bei den mittels Roll-on-Vorrichtungen auftragbaren Mitteln dagegen einen solchen von 3,0 bis 5,5 Gewichts-%, jeweils bezogen auf die Gesamt-Zusammensetzung des Mittels.

Als übliche kosmetische Trägerstoffe zur Herstellung der desodorierenden Mittel gemäß der Erfindung können neben Äthanol und Isopropanol, Glycerin und Propylenglykol hautpflegende Fett- oder fettähnliche Stoffe, wie Partialglyceride von Fettsäuregemischen, Ölsäuredecylester, Cetylalkohol, Cetylstearylakohol und 2-Octyldodecanol, in den für solche Präparate üblichen Mengenverhältnissen eingesetzt werden sowie schleimbildende Stoffe und Verdickungsmittel, wie Methylcellulose, Polyacrylsäure, Polyvinylpyrrolidon. Daneben aber auch in kleinen Mengen cyclische Silikonöle (Polydimethylsiloxane) sowie flüssige Polymethylphenylsiloxane niedriger Viskosität.

Zwecks Erzielung einer verstärkten desodorierenden Wirkung oder falls besondere Einsatzbedingungen dieses erfordern, können den kosmetischen Mitteln gemäß der Erfindung zusätzlich 0,1 bis 0,2 Gewichts-%, bezogen auf die Gesamt-Zusammensetzung, eines bekannten antibakteriell wirksamen Mittels, wie 2,4,4'-Trichlor-2'-hydroxydiphenyläther oder Undecylensäure-polydiäthanolamid, zugesetzt werden.

Darüber hinaus können den Mitteln 5,0 bis 10,0 Gewichts-%, bezogen auf die Gesamt-Zusammensetzung, an bestimmten, im alkoholischen oder wässrig-alkoholischen Milieu beständigen Aluminiumverbindungen, wie Aluminiumchlorhydrat, Natriumaluminiumchlorhydroxylactat ("Chloracel") oder Aluminiumhydroxychlorid-Propylenglykol-Komplex-Verbindungen ("Rehydrol ASC") zugesetzt werden. Anstelle einer der vorstehend genannten Wirkstoffe kann - falls dies gewünscht wird - auch ein Gemisch aus zwei dieser Substanzen eingesetzt werden. Beim Einsatz dieser Substanzen in solchen Mitteln, die aus Aerosolbehältern mittels eines verflüssigten Treibmittels versprüht werden sollen, ist im Hinblick auf mögliche Wirkstoffveränderungen und Korrosionsprobleme Wasserfreiheit anzustreben.

Als Treibmittel für aus Aerosolbehältern in Form eines Sprühstrahls bei Betätigung des Ventils versprühbare desodorierende kosmetische Mittel gemäß der Erfindung sind die üblichen bekannten leichtflüchtigen, verflüssigten Treibmittel, wie Kohlenwasserstoffe (Propan, Butan, Isobutan) und chlorfluorierte Kohlenwasserstoffe (Dichlordifluormethan, Trichlormonofluormethan, Dichlortetrafluoräthan) geeignet, die allein oder in Mischung miteinander eingesetzt werden können.

Als Emulgatoren zur Herstellung der erfindungsgemäßen desodorierenden kosmetischen Mittel, die vorzugsweise als flüssige Zubereitungen mittels einer Roll-on-Vorrichtung auf die gewünschten Hautbereiche aufgetragen werden sollen, und die in den Mitteln in geringer Menge (2 bis 5 Gewichts-%, bezogen auf die Gesamt-Zusammensetzung) verwendet werden können, haben sich nichtionogene Typen, wie Polyoxyäthylen-Fettalkoholäther, z.B. Cetostearylalkoholpolyäthylenglykoläther mit 12 bzw. 20 angelagerten Äthylenoxid-Einheiten pro Molekül Cetostearylalkohol, sowie Sorbitanester und Sorbitanester-Äthylenoxid-Verbindungen (z.B. Sorbitanmonostearat und Polyoxyäthylensorbitanmonostearat) als auch langkettige höhermolekulare wachsartige Polyglykoläther als besonders geeignet erwiesen.

Zusätzlich zu den genannten Bestandteilen können den desodorierenden kosmetischen Mitteln gemäß der Erfindung, deren pH-Wert vorzugsweise auf 6,0 - 7,5 eingestellt wird, kleine Mengen Parfüm, Farbstoffe, Antioxidantien (z.B. "Ionol"=2,6-Di-tert.-butyl-4-methylphenol in Mengen von 0,01 bis 0,03 %, bezogen auf die Gesamt-Zusammensetzung), Suspendiermittel, Komplexbildner, Puffergemische oder andere übliche kosmetische Grundstoffe, wie Triäthanolamin oder Harnstoff, beigemischt werden.

Zur Parfümierung sind insbesondere solche Substanzen und Parfümöle geeignet, die stabil sind, die Haut nicht reizen und bereits als solche antibakterielle (bakteriostatische) Eigenschaften besitzen.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Die Herstellung der kosmetischen Mittel erfolgt abgesehen von speziellen Zubereitungen, die in den Beispielen jeweils gesondert vermerkt sind, in üblicher Weise, zumeist durch einfaches Vermischen unter leichtem Rühren, gegebenenfalls unter leichter Erwärmung. Sie bietet keine Schwierigkeiten.

Die Erfindung wird anschließend anhand von Beispielen näher erläutert. Die gemäß den folgenden Beispielen hergestellten Präparate zeigten in jedem Falle neben einer ausgezeichneten Haut- und Schleimhautverträglichkeit bei bestimmungsgemäßer Anwendung eine sehr gute und lang anhaltende desodorierende Wirkung.

## Beispiel 1

Alkoholischer Deodorant-Spray

|  | Gew.Teile |
|---|---|
| Wollwachssäuren | 0,3 |
| Äthanol, abs. | 35,5 |
| Isopropanol | 3,7 |
| Parfüm | 0,5 |

Die aufgeführten Bestandteile wurden unter leichtem Rühren bis zum Erhalt einer klaren Lösung auf 50°C erwärmt. Die erhaltene Lösung wurde in eine Aerosol-Dose gefüllt. Nach dem Verschließen der Dose wurden über das Ventil 60 Gew.-Teile eines Treibmittelgemisches aus Dichlordifluormethan (Frigen 12) und Trichlormonofluormethan (Frigen 11) im Verhältnis 50 : 50 aufgepreßt.

## Beispiel 2

Alkoholischer Deodorant-Spray

|  | Gew.-Teile |
|---|---|
| Wollwachssäuren | 0,5 |
| Äthanol, abs. | 35,3 |
| Isopropanol | 3,7 |
| Parfüm | 0,5 |

Die Herstellung des Präparats erfolgte entsprechend Beispiel 1.

## Beispiel 3

Deodorant-Emulsion (O/W) für Roll-on-Vorrichtungen

|  | Gew.-Teile |
|---|---|
| Gemisch von Mono- und Diglyceriden der Palmitin- und Stearinsäure | 4,0 |
| Umsetzungsprodukt aus 12 Molekülen Äthylenoxid und eines techn. Gemisches von nativem Cetyl- und Stearylalkohol | 1,5 |

| | Gew.-Teile |
|---|---|
| Umsetzungsprodukt aus 20 Molekülen Äthylenoxid und eines techn. Gemisches aus nativem Cetyl- und Stearylalkohol | 1,5 |
| 2-Octyldodecanol | 5,0 |
| Natriumsalz der Wollwachssäuren | 5,5 |
| Äthanol, 96 %ig | 10,0 |
| Acrylsäurepolymerisat ("Carbopol 934") | 0,3 |
| Triäthanolamin | 0,5 |
| Glycerin, 86 %ig | 5,0 |
| Wasser (voll entsalzt) | 66,7 |

Die Fettstoffe wurden auf dem Wasserbade bei etwa 70°C geschmolzen. Getrennt davon wurden 50 Teile des Wassers mit Glycerin vermischt und ebenfalls auf etwa 75°C erhitzt. Dieser Wasserphase wurde die geschmolzene Fettphase bei der angegebenen Temperatur langsam unter Rühren zugefügt. Das Acrylsäurepolymerisat wurde in den restlichen Wasseranteilen unter Rühren aufgequollen. Nach Abkühlung der sich nach Vereinigung der Fettphase mit der Wasserphase gebildeten Emulsion auf etwa 35°C wurde dieser das Äthanol und die Acrylsäurepolymerisat-Quellung zugefügt, parfümiert und anschließend mit Triäthanolamin neutralisiert.

## Beispiel 4

<u>Deo-Lotion</u>  (zum Einsatz in Form von Quetschflaschen oder aus Behältern mit Pumpvorrichtung)

| | Gew.-Teile |
|---|---|
| Umsetzungsprodukt aus 25 Molekülen Äthylenoxid und eines techn. Gemisches aus nativem Cetyl- und Stearylalkohol | 2,0 |
| Cetylstearylakohol | 3,0 |
| Paraffinöl | 5,0 |
| 1,2-Propylenglykol | 3,0 |
| Polyvinylpyrrolidon ("Luviskol K30", BASF) | 0,5 |

| | Gew.-Teile |
|---|---|
| Natriumsalz der Wollwachssäuren | 5,0 |
| Wasser (voll entsalzt) | 81,5 |

Die Herstellung des Präparats erfolgte analog zu Beispiel 3.

## Beispiel 5

### Roll-on-Deodorant-Lotion

| | Gew.-Teile |
|---|---|
| Polyäthylenglykol 400 | 3,0 |
| Natriumsalz der Wollwachssäuren | 4,8 |
| Äthanol, abs. | 30,0 |
| Kamillenextrakt, glykolisch | 2,0 |
| Methylcelluloseschleim 5 %ig | 44,7 |
| Aluminiumchlorhydrol 50 %ige wässr. Lsg. | 15,0 |
| Parfümöl | 0,5 |

## Beispiel 6

### Roll-on-Deodorant-Lotion

| | Gew.-Teile |
|---|---|
| Gemisch von Mono- und Diglyceriden höherer Fettsäuren, vornehmlich Palmitin- u. Stearinsäure ("Cutina MD", Henkel) | 1,0 |
| Cetylstearylalkohol | 3,0 |
| 2-Octyldodecanol | 5,0 |
| Wollwachssäuren | 4,0 |
| 1,2-Propylenglykol | 16,0 |
| cycl. Silikonöl ("Pentamer") | 3,0 |
| Wasser (voll entsalzt) | 65,8 |
| Hydroxyäthylcellulose-Schleim 3%ig | 2,0 |
| Parfüm | 0,2 |

## Beispiel 7

Roll-on-Deodorant-Lotion

|  | Gew.-Teile |
|---|---|
| Palmitinsäurediglycerid | 2,0 |
| Cetylstearylalkolhol | 3,0 |
| 2-Octyldodecanol | 5,0 |
| Wollwachssäuren | 5,0 |
| Triäthanolamin | 0,45 |
| 1,2-Propylenglykol | 16,0 |
| cycl. Silikonöl ("Pentamer") | 3,0 |
| Wasser (voll entsalzt) | 64,25 |
| Polyacrylsäureschleim 2 %ig | 1,0 |
| Parfüm | 0,3 |

Zur Herstellung der Präparate gemäß den Beispielen 6 und 7 wurden die Fettstoffe bei 75°C geschmolzen und die getrennt davon hergestellte wässrige Phase, bestehend aus Triäthanolamin (Beispiel 7), Propylenglykol, Silikonöl und Wasser, wurde auf 65°C erhitzt und zur geschmolzenen Fettphase langsam unter Rühren zugegeben. Bei etwa 40°C wurde alsdann die Schleimstoff-Zubereitung (Verdickerschleim) und bei etwa 30°C das Parfüm zugefügt. Die Homogenisierung des erhaltenen Produktes erfolgte über eine übliche Homogenisiermaschine, beispielsweise über eine Homocenta in Stufe 2.

Patentansprüche

1. Desodorierendes kosmetisches Mittel auf Basis üblicher kosmetischer Trägerstoffe sowie ein oder mehrerer Wirkstoffe, dadurch gekennzeichnet, daß es als Wirkstoff Wollwachssäuren oder Alkali- oder Ammoniumsalze von Wollwachssäuren enthält.

2. Desodorierendes Mittel nach Anspruch 1, dadurch gekennzeichnet, daß es Wollwachssäuren oder deren Alkali- oder Ammoniumsalze in einer Menge von 0,3 bis 6,0 Gewichts-%, bezogen auf die Gesamt-Zusammensetzung des Mittels, enthält.

3. Desodorierendes Mittel nach den Ansprüchen 1 oder 2, dadurch gekennzeichnet, daß es in Form eines aus Aerosolbehältern, Quetschflaschen oder durch eine Pumpvorrichtung versprühbaren Präparates vorliegt.

4. Desodorierendes Mittel nach den Ansprüchen 1 oder 2, dadurch gekennzeichnet, daß es in Form eines mittels Roll-on-Vorrichtungen auftragbaren Präparates vorliegt.

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.³) |
|---|---|---|---|
| X | DE-A- 647 451 (DR. HENRYK COHN et al.) <br> * Seite 2; Beispiel * | 1 | A 61 K 7/32 |
| X | FR-A-2 268 516 (PROCTER & GAMBLE) <br> * Seite 11; Patentansprüche * | 1,2 | |
| X | FR-A-2 381 558 (L'OREAL) <br> * Seite 11; Patentanspruch 1 * | 1 | |
| X | FR-A-2 132 130 (L'OREAL) <br> * Seite 14; Patentanspruch 1 * | 1 | |
| Y | FR-A-2 035 901 (J.B. WILLIAMS CO.) <br> * Seite 12; Patentansprüche * | 1 | RECHERCHIERTE SACHGEBIETE (Int. Cl.³) |
| P,X | EP-A-0 081 721 (BEIERSDORF A.G.) <br> * Patentansprüche; Seite 2, Zeile 29 - Seite 3, Zeile 3 * | 1 | A 61 K |

Der vorliegende Recherchenbericht wurde für alle Patentansprüche erstellt.

| Recherchenort <br> DEN HAAG | Abschlußdatum der Recherche <br> 12-09-1984 | Prüfer <br> STIENON P.M.E. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

 

& : Mitglied der gleichen Patentfamilie, übereinstimmendes Dokument

EPA Form 1503. 03.82